# EUROPEAN PATENT APPLICATION

(11) **EP 4 631 489 A2**
(43) Date of publication of application: **15.10.2025**
(21) Application number: 25199181.6
(22) Date of filing: 30.08.2021
(51) Int. Cl.: A61J 7/00

(54) **METHODS OF PREPARING MODIFIED DOSAGE FORMS AND RELATED COMPONENTS**

(30) Priority: 01.09.2020 US 202063073049 P; 30.03.2021 US 202163167988 P
(62) Divisional of application: 21864952.3
(71) Applicant: The Global Alliance for TB Drug Development, Inc., New York, NY 10005 (US)
(72) Inventor: TANEJA, Rajneesh, New York, 10025 (US); SCARIM, Joseph Anthony, Tucson, 85750 (US)
(74) Representative: Plasseraud IP

(57) **Abstract**

Provided are methods of preparing a homogeneous mixture from a solid dosage form in settings including a point of care setting. The methods include obtaining a solid dosage form comprising a drug product, adding the solid dosage form to a container having at least one flexible section, adding a liquid to the container, mixing the solid dosage form with the liquid to disperse, disintegrate, suspend, and/or dissolve the solid dosage form thereby creating a homogeneous mixture. Also provided are containers and devices for use in such methods.

## Description

### CROSS-REFERENCE TO RELATED APPLICATIONS

This application claims priority of U.S. Provisional Application No. 63/073,049, filed September 1, 2020, and U.S. Provisional Application No. 63/167,988, filed March 30, 2021, the contents of each of which are hereby incorporated by reference in their entirety.

### FIELD OF THE INVENTION

This invention is directed to, for example, methods of converting solid dosage forms to homogeneous mixtures in a point of care setting and other settings.

### BACKGROUND OF THE INVENTION

Solid dosage forms are the default standard for pharmaceutical drug formulations despite the fact that a significant percentage of the population needs to take partial doses of solid dosage forms and/or are unable to swallow tablets and capsules. The present invention provides methods to address the shortcomings of solid dosage forms in these cases.

### SUMMARY OF THE INVENTION

Provided is a method of preparing a homogeneous mixture from a solid dosage form comprising
a) obtaining a solid dosage form comprising a drug product,
b) adding the solid dosage form to a container,
c) adding a liquid to the container,
d) mixing the solid dosage form with the liquid to disperse, disintegrate, suspend, and/or dissolve the solid dosage form thereby forming the homogeneous mixture;
wherein the container comprises at least one flexible section and the method is performed at a point of care of a subject.

Also provided is a method of preparing a homogeneous mixture from a solid dosage form comprising
a) obtaining a solid dosage form comprising a drug product,
b) adding the solid dosage form to a container,
c) adding a liquid to the container,
d) mixing the solid dosage form with the liquid to disperse, disintegrate, suspend, and/or dissolve the solid dosage form thereby forming the homogeneous mixture;

wherein the container comprises at least one flexible section and the homogeneous mixture is intended to be administered to a subject,
wherein (a) the subject is a non-human animal or a human, or (b) the amount of the drug product administered or to be administered to the subject is determined by characteristics of the subject, or (c) one or more additional solids dosage forms are added to the container, or (d) the subject is participating in a clinical trial, or (e) the subject is a pediatric subject, or (f) the subject is a geriatric subject.

### BRIEF DESCRIPTION OF THE DRAWINGS

The drawings described below are for illustrative purposes only and are not intended to limit the scope of the invention.
Figures 1, 2 and 3 depict disposable bags which may be used in the methods of the present invention.
Figure 4A depicts a container which may be used in the methods of the present invention. Figures 4B and 4C depict support tubes which may be used in the methods of the present invention.
Figure 5 depicts a container which may be used in the methods of the present invention.
Figure 6A depicts a container which may be used in the methods of the present invention. Figures 6B and 6C depict the same container depicted in Figure 6A connected to an amber prescription bottle.
Figures 7 and 8 depict containers which may be used in the methods of the present invention.
Figure 9 depicts a container and syringe adapter which may be used in the methods of the present invention.

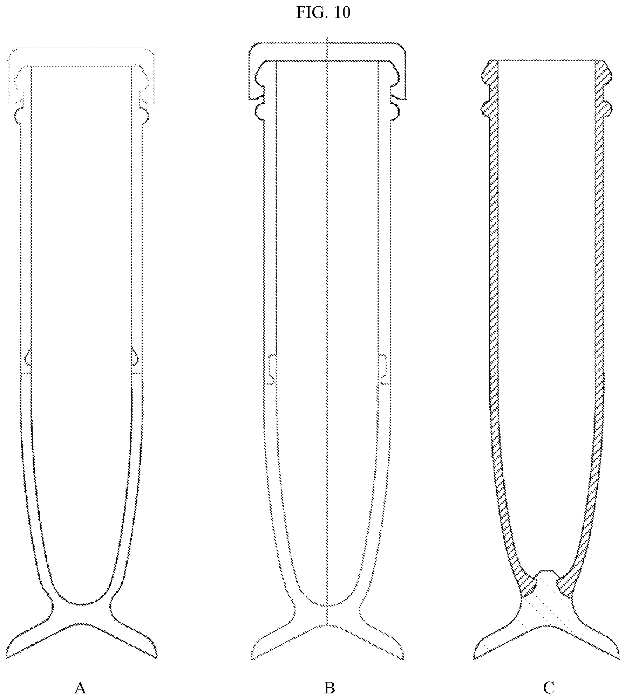

Figure 10 depicts containers with detachable pieces which may be used in the methods of the present invention.
Figure 11 depicts a container and tablet which may be used in the methods of the present invention.
Figure 12 depicts a container and a vibrating device which may be used in the methods of the present invention.
Figure 13 depicts a container and a device which may be used in the methods of the present invention.
Figure 14 depicts a container with an electronic device which may be used in the methods of the present invention.

### DETAILED DESCRIPTION OF THE INVENTION

It is to be understood that the terminology employed herein is for the purpose of describing particular embodiments, and is not intended to be limiting. Further, although any methods, devices and materials similar or equivalent to those described herein can be used in the practice or testing of the invention, certain methods, devices and materials are now described.

For the foregoing embodiments (including elements recited in the number paragraphs), each embodiment disclosed herein is contemplated as being applicable to each of the other disclosed embodiments. In addition, the elements recited in the method embodiments can be used in the container, powder blend or system embodiments described herein.

### Definitions

The articles "a" and "an" are used in this disclosure to refer to one or more than one (i.e., to at least one) of the grammatical object of the article.

A "subject" may be a human or a non-human animal. The terms "subject" and "patient" are used interchangeably herein.

The term "treating," with regard to a subject, encompasses, e.g., inducing inhibition, regression, or stasis of a disease or disorder; or curing, improving, or at least partially ameliorating the disorder; or alleviating, lessening, suppressing, inhibiting, reducing the severity of, eliminating or substantially eliminating, or ameliorating a symptom of the disease or disorder. "Inhibition" of disease progression or disease complication in a subject means preventing or reducing the disease progression and/or disease complication in the subject.

A "symptom" associated with a disease or disorder includes any clinical or laboratory manifestation associated with the disease or disorder and is not limited to what the subject can feel or observe.

"Administering to the subject" or "administering to the patient" means the giving of, dispensing of, or application of medicines, drugs, or remedies to a subject/patient to relieve, cure, or reduce the symptoms associated with a condition, e.g., a pathological condition. The administration can be periodic administration.

As used herein, "effective" or "therapeutically effective" when referring to an amount of a substance, for example a drug, refers to the quantity of the substance that is sufficient to yield a desired therapeutic response. In certain embodiments, an effective amount refers to an amount effective, at dosages and for periods of time necessary, to achieve the desired therapeutic or prophylactic result. A therapeutically effective amount of a drug of the invention may vary according to factors such as the disease state, age, sex, and weight of the individual, and the ability of the drug to elicit a desired response in the individual. A therapeutically effective amount encompasses an amount in which any toxic or detrimental effects of the drug are outweighed by the therapeutically beneficial effects.

By any range disclosed herein, it is meant that all hundredth, tenth and integer unit amounts within the range are specifically disclosed as part of the invention. Thus, for example, 0.01% to 50% means that 0.02, 0.03 ... 0.09; 0.1; 0.2 ... 0.9; and 1, 2... 49 % unit amounts are included as embodiments of this invention.

As used herein, a "homogeneous mixture" is a mixture in which the composition is approximately uniform throughout the mixture. For example, a homogeneous mixture consisting of water and a drug product, after being divided into two parts of equal volume or weight, may have the same amount of the drug product, or about the same amount of drug product. The two parts may have about the same amount of drug product if, for example, the difference is 1% or less (at least 99% homogenous), 1.5% or less (at least 98.5% homogeneous), 2% or less (at least 98% homogeneous), 5% or less (at least 95% homogeneous), or 10% or less (at least 90% homogeneous). Thus, in embodiments of the invention, the homogeneous mixture is at least 90% homogeneous, at least 95% homogeneous, at least 98% homogeneous, at least 98.5% homogeneous or at least 99% homogeneous.

Homogeneous mixtures may become heterogeneous after a period of time. A homogeneous mixture is homogeneous immediately after a mixing step. In embodiments, the mixture is homogenous for at least: 1 minute, 2 minutes, 5 minutes, 10 minutes, 30 minutes, or 1 hour, after the mixing step. Additionally, a mixture or suspension may be considered homogeneous in some embodiments if it was homogeneous at an earlier point of time, and can be made homogenous by briefly mixing or shaking the mixture or suspension.

It is understood that a solid may be simultaneously dissolved, dispersed, suspended and/or disintegrated into a liquid such that some portions of the solid are dissolved in the liquid, some portions are dispersed into the liquid, some portions are suspended into the liquid and some portions are disintegrated into the liquid. For example, a solid that is dispersed into a liquid may also have some portion of the solid dissolved and/or disintegrated into the liquid.

As used herein, "homogeneous mixture intended to be administered to a subject" means that the homogeneous mixture is intended to be or capable of being administered to a subject at a later point in time or at multiple points of time in the future. This definition includes a homogeneous mixture prepared with a possibility of being administered that are not actually administered. In some embodiments, the method further comprises administering the homogeneous mixture to the subject.

In embodiments, the homogeneous mixture is a homogeneous suspension.

The term "container" is used herein broadly. Thus, a container includes, but is not limited to, bags, pouches, vessels, housings, tubes, receptacles, dosing aids, devices, and the like.

In some embodiments, the container is capable of standing upright. In one such embodiment, a suction cup is incorporated into the bottom of the container. This suction cup provides support.

In some embodiments, the bottom of the inside of the container is hemispherical, tapered or conical. A hemispherical, tapered or conical bottom prevents or minimizes accumulation of solids at the bottom of the container. For example, a container with corners would have solids accumulate in the corners. Additionally, the hemispherical, tapered or conical bottom allows sufficient contact with the tablet during the mixing step. Such contact allows the solid dosage form to disintegrate faster.

In embodiments, the container comprises a means for reversibly enclosing and sealing the container. The means for reversibly enclosing and sealing the container may be a cap. Such a feature permits storage of the prepared suspension for future use. The means for reversibly enclosing and sealing the container is preferably leak proof or minimizes leaks. Storage of the container and homogeneous mixture or suspension in cold temperatures, such as in a freezer or refrigerator, is aided by the means for reversibly enclosing and sealing the container. The means for reversibly enclosing and sealing the container may be child-resistant.

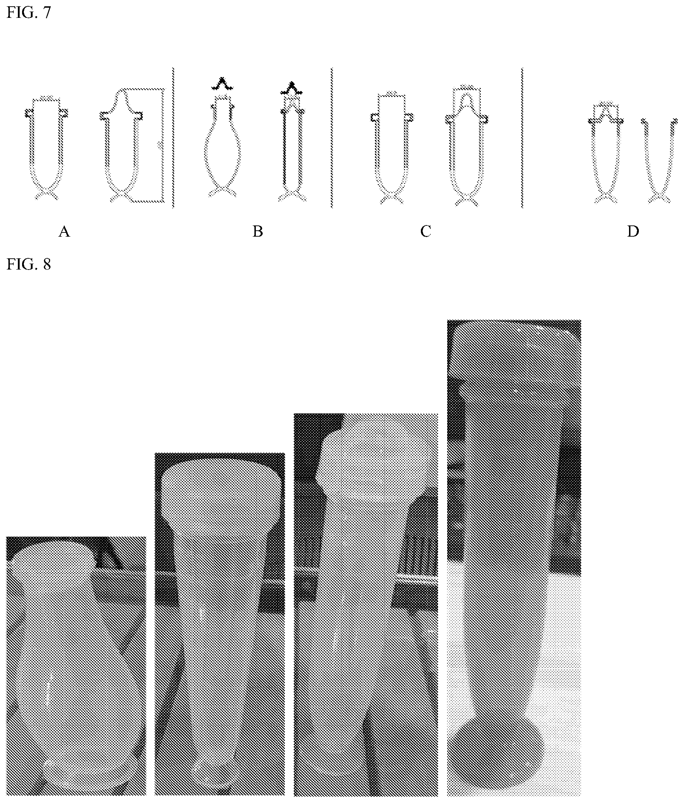

Exemplary non-limiting embodiments are shown in Figure 7. The container may be shaped to maximize appeal to a particular audience. For example, the outside of the container may be shaped as an animal, for example as a cartoon character, to appeal to children. The internal design may remain unchanged from the other embodiments described herein.

In some embodiments, the method comprises administering, or having administered, the homogeneous mixture to the subject directly from the opening of the container to the oral cavity of the patient.

The container may comprise an elongated tapering neck. The neck can have graduation markings to indicate the volume dispensed. The container can be inverted and squeezed to dispense the desired quantity directly to the patient.

In some embodiments the means for reversibly enclosing and sealing the container, e.g., the cap, comprises an oral spoon. In some embodiments, the means for reversibly enclosing and sealing the container comprises a syringe adapter or is designed to accommodate a syringe adapter. Such a syringe adapter permits an oral syringe tip to connect to the container and allows for the accurate withdrawal of the homogeneous mixture from the container. As such, the challenges with pouring the liquid or dipping a syringe into the container are eliminated.

In some embodiments, the container comprises an external handle. Such a handle permits holding and manipulating the container with one hand allowing use of the other hand for holding another device such as a dosing cup or a dosing spoon.

In embodiments, the container comprises an inner side and an outer side, wherein the inner side and/or the outer side comprises bumps, a coarse texture, protrusions, rings, spirals, dots, raised surfaces and/or ridges. Such features are useful for one to securely hold the container (e.g., preventing the container from slipping) or for facilitating the disintegration of the solid dosage form. The inner side is typically the side which is in contact with the liquid or homogeneous mixture.

In some embodiments, the container comprises at least two parts, a first part having a first chamber, first opening and a second opening, and a second part having a second chamber and a third opening, wherein the second opening is capable of attaching to and forming a seal with the third opening such that the first and second chambers form a larger single chamber. For example, the container may have a bottom section and a top section which are reversibly attachable and detachable. Such features provide benefits in the blending of solid dosage forms and removing homogeneous mixtures.

In embodiments, the container is lined with a bag that fits inside the container. This bag (e.g. a second container) can be disposed of after preparing and dispensing the dose. This container can thus be used without washing and cleaning.

In embodiments, the device or container has color incorporated into its body or is wrapped with colored or opaque material such as a label. This can increase the stability of photolabile drugs during storage.

The container or device can be sterilized by using gas, radiation or any other conventional methods.

In some embodiments, the container is thin, pliable, and flexible and comprises, or is made of, a plastic film, nonwoven fabric, or plastic textile. The plastic film, nonwoven fabric, or plastic textile may be polyethylene, for example, high-density polyethylene (HDPE), low-density polyethylene (LDPE), or linear low-density polyethylene (LLDPE), polyethylene terephthalate (PET), or polypropylene, or other pliable polymers, or mixtures thereof.

In some embodiments, the container comprises, or is made of, silicone, a polycarbonate or polystyrene.

In embodiments, the container may be used concurrently with a housing or a second container having a rigid body. The container may be inserted into the rigid housing or second container. Alternatively, the rigid housing or second container may be inserted into the container to act as a support insert tube. The container may be discarded after a single use while the rigid housing or second container may be rinsed or washed and reused.

In some embodiments, the opening of the container is defined by opposed side edges capable of being reversible coupled together. **In** some embodiments, the opening consists of a pair of complementarily opposed seal strips, one strip comprising a vane component, and the second strip comprising a channel component, and when such strips are engaged, form a liquid impermeable barrier. One such example is a container having a zip-top. The container may include a squeeze open mouth to allow for easy insertion of solid dosage form and the drug product into the container without inserting of a solid support. The squeeze open mouth may be accomplished by using a stiffer material or polymer for the opening. For example, a HDPE may be used.

In embodiments, the solid dosage form is mixed, dispersed, disintegrated, suspended and/or dissolved by force. The term "force" is used herein broadly. Thus, force includes, but is not limited to, agitation, vibration, shaking, rubbing, mechanical, pressure or pressing, shearing, striking, bouncing, abrasion and/or erosion. The above list may include terms with overlapping definitions. For example, some vibrational forces would also be considered shaking forces and vice versa.

In embodiments, mixing comprises applying a force to the container. In embodiments, the force may be a vibrational force, a shaking force, a mechanical force, a rubbing force, and/or mechanical pressure to the flexible section of the container. The force may be applied by a human. Alternatively, the force may be applied by a machine. In embodiments, the machine comprises, a shaking mechanism, a vibrating mechanism, a mechanism to provide mechanical force, a mechanism to provide rubbing force, or a mechanism to provide mechanical pressure.

Also provided is a system comprising a container as described herein and a machine capable of providing a force to the container sufficient to disintegrate a solid dosage form. The machine may be an electronic device.

In embodiments, the container is inserted into a chamber of the machine. In embodiments, the machine applies mechanical force to the container by one or more rollers or pistons. The force may be in the form of an oscillatory motion and/or a rolling motion.

In one embodiment, the device or machine contains one or more rollers capable of exerting force on the container (and the solid dosage form therein) to aid in disintegrating the solid dosage form. In one embodiment, the device or machine contains two rollers capable of exerting force on the container (and the solid dosage form therein) to aid in disintegrating the solid dosage form. In some embodiments, the device or machine contains a means for increasing or decreasing the distance between the rollers. In some embodiments, the rollers moves and the container is stationary. In other embodiments, the container moves and the rollers are stationary but may still rotate. The rotation of the roller(s) may be controlled by an electric motor or manually by a mechanical means, for example by a lever that can be rotated by a person which in turn rotates the roller(s). The rotation of the roller(s) may also be caused by movement of the container.

Multiple forces may be applied to the container (and the solid dosage form therein). All combinations of the disclosed forces are contemplated. For example, the following combination of forces may be applied:
a) vibrational and mechanical forces;
b) vibrational, shaking, and mechanical forces;
c) vibrational, shaking, rubbing and mechanical forces;
d) vibrational, shaking, rubbing, pressure and mechanical forces;
e) shaking and mechanical forces;
f) vibrational, shaking, and mechanical forces;
g) shaking and mechanical forces;
h) rubbing and mechanical forces;
i) rubbing, shaking, and mechanical forces;
j) pressure and mechanical forces;
k) pressure, shaking, and mechanical forces;
l) pressure, shaking, rubbing and mechanical forces;
m) shaking and vibrational forces;
n) shaking, rubbing and vibrational forces; and
o) shaking, rubbing, pressure and vibrational forces.

In embodiments, the forces are applied as in any one of subsections a) to o) above. In another embodiment, a rolling force is applied in addition to any of one of the forces of subsections a) to o). In a further embodiment, a pressing force is applied in addition to any of one of the forces of subsections a) to o).

The mechanical force may be provided by any suitable means, for example, by human hands, rollers, or pistons.

The different forces may be applied concurrently or sequentially or in any order suitable to dissolve, disperse, suspend and/or disintegrate the solid dosage form. In embodiments, the container is in the horizontal configuration. This facilitates rolling the container on a solid surface to allow for rubbing and other forces thereby breaking up the tablet.

In some embodiments, the container comprises a section with a thinner wall thickness and a second section with a thicker wall thickness. For example, the bottom section of the container may have a thickness of 1-1.5 mm and the upper section may have a thickness of 2mm or higher. This provides a section of the container that allows for a person to more easily massage and break up the dosage form.

In some embodiments, the water is deionized water or distilled water.

In some embodiments, subject is a human, a non-human animal, participating in a clinical trial, a pediatric subject, and/or a geriatric subject.

In some embodiments, a preservation agent is used in the methods of the present invention, for example in the powder blends. One or more preservation agents may be added to the container containing the drug product and liquid. The preservation agent may decrease or lower the oxidation of the active ingredients or excipients and reduce microbial production. Some exemplary preservation agents include antibacterial agents, antifungal agents, antimicrobial agents, microstatic agents, microbicidal agents, phenol alcohol, benzyl alcohol, phenoxyethanol, butyl paraben, chlorobutanol, meta cresol, chlorocresol, methyl paraben, phenyl ethyl alcohol, propyl paraben, phenol, benzoic acid, sorbic acid, methyl paraben, sodium benzoate, bronidol, and propylene glycol.

In some embodiments, a viscosity modifier or thickener is used in the methods of the present invention, for example in the powder blends. One or more viscosity modifiers may be added to the container containing the drug product and liquid. Some exemplary viscosity modifiers include guar gum, cellulose gel, povidone, benzyl alcohol, polyethylene glycol, a blend of esters of behenic acid and glycerol, glyceride, a mixture of ethylene glycol palmitostearate and diethylene glycol palmitostearate, hydroxypropyl cellulose, maltodextrins and dried glucose syrups, sodium starch glycolate, pregelatinized maize starch, and hydrocolloids.

In some embodiments, an anti-foaming agent may be used into the methods of the present invention, for example in the powder blends. One or more anti-foaming agents may be added to the container containing the drug product and liquid. Some exemplary anti-foaming agents include bisphenylhexamethicone, dimethicone, hexamethyldisiloxane, dimethiconol, hexyl alcohol, isopropyl alcohol, tetramethyl decynediol, simethicone (a mixture of about 90% dimethicone and 10% silicone dioxide (w/w)), phenethyl disiloxane, phenyl trimethicone, petroleum distillates, polysilicone-7, propyl alcohol, silica dimethyl silylate, silica silylate, and trimethylsiloxysilicate. In embodiments, the anti-foaming agent is an alcohol (for example, cetostearyl alcohol), insoluble oils (for example, castor oil), stearates, polydimethylsiloxanes and other silicones derivatives, ether or glycols.

In some embodiments, the drug product may be, for example, metoclopramide, zolpidem, ondansetron, amlodipine, atorvastatin, venlafaxine, carvedilol, salbutamol sulphate, fexofenadine, metoprolol, metformin, cefixime, irbesartan, divalproex, acyclovir, rosuvastatin, almotriptan, cinnarizine, dimenhydrinate, repaglinide, telmisartan, levonorgestrel, bisoprolol, fexofenadine, aripiprazole, amphetamine, loratadine, donepezil, diphenhydramine, paracetamol, escitalopram, desloratadine, desmopressin, etizolam, clozapine, n-acetylcysteine, acetaminophen, baclofen, clonazepam, lamotrigine, rizatriptan, meloxicam, alprazolam, hyoscyamine, ibuprofen, prednisolone, carbidopa, levodopa, famotidine, lansoprazole, cisapride, mirtazapine, risperidone, tramadol, asenapine, vardenafil, testosterone, buprenorphine naloxone, phentermine, diphenhydramine, domperidone, selegiline, zolmitriptan, olanzapine, cetirizine, moxifloxacin, doxycycline, trimethoprim, paracetamol, amikacin, capreomycin, cycloserine, ethionamide, kanamycin, levofloxacin, linezolid, p-aminosalicylic acid, or streptomycin. Other examples of drug products may be found in the WHO Model List of Essential Medicines for Children (April 2015) available at: www.who.int/medicines/publications/essentialmedicines/EMLc2015_8-May-15.pdf?ua=1 which is hereby incorporated by reference.

The drug in the drug product may be in any suitable form including, but not limited to, a pharmaceutically acceptable salt of the drug or a derivative of the drug. The term "pharmaceutically acceptable salt" in this respect, refers to the relatively non-toxic, inorganic and organic acid or base addition salts of the drug. For example, one means of preparing such a salt is by treating a drug with an inorganic base.

In some embodiments, the method comprises adding a second drug product to the container, optionally in step b) of the method. The second drug product may be a second solid dosage form or a second drug may be in the same solid dosage form as the first drug. In other embodiments, more than two drug products are added to the container, optionally in step b) of the method.

Any medium molecular weight methylcellulose thickener may be used in the present methods and formulations, for example in the powder blends, including Methocel^{™} A4C Premium.

In some embodiments, the container is sterilized. The container may be provided in a sterilization pouch.

The subject disclosure permits dosing via feeding tubes, for example, in a hospital setting. The disclosure also permits the accurate dosing of partial doses from a solid dosage form. Such partial dosages may be stored if stability data permits.

The challenge of administering certain oral dosage forms to patients with swallowing difficulties has existed for many decades. Multiple devices are known in the art. However, the herein disclosed methods present a unique solution which is not presently practiced, for example, by health care professionals or in other areas such as veterinary medicine, precision medicine, patient compliance, and clinical trials.

Powder blends are described herein which thicken upon the contact with water to provide desired viscosity. This helps in making and sustaining a homogeneous suspension. Thickening and viscosity must be controlled in this process and the formulations described herein allow for proper control. The formulations described herein also provides good taste for patients and good organoleptic properties.

Many oral tablets are typically ingested along with 240 ml of water to ensure that they disintegrate and subsequently undergo dissolution in the gastrointestinal tract. During the dissolution testing of these tablets, 900 mL of aqueous media is required. The present method allows for disintegrating and dispersing these tablets uniformly in 5-15 ml of water or other liquid. An apparatus is utilized that permits mixing and ensuring that a homogeneous dispersion is formed. This apparatus utilizes the principle of churning and mixing to mimic the stomach during digestion. During the use of this apparatus, the viscosity of the suspension increases gradually to permit water penetrating into the tablet.

The disclosed invention has many different applications including, but not limited to, point of care, veterinary, precision medicine, compliance enhancement, and clinical trials. Thus, in some embodiments, the method is applicable to, or practiced in a point of care setting, a veterinary setting, precision medicine, compliance enhancement or a clinical trial setting.

Administering solid dosage forms to pets and livestock can be facilitated by using the methods and devices disclosed herein. The tablets can be converted to a suspension. These suspensions may be flavored with meat and fish flavors or be mixed with pet food. Different animals and species have different body masses and other characteristics and thus require varying amounts of active ingredients. The methods and devices disclosed herein permit the use of appropriate doses. Larger devices and liquid amounts (e.g., 1 liter, 500 ml, or 100 ml) allow for dose preparation on a larger scale. For example, elephants have to be fed a number of drugs in large doses for treating tuberculosis.

There is incongruity between drug development and clinical medicine in how to tackle variability in drug response. Drug development has a one-dose-fits-all culture because it is impractical and cost prohibitive to study too many different doses and too many different types of patients. Precision dosing is set to become more sophisticated. Affordable omics technologies (e.g., genomics, transcriptomics, proteomics, and metabolomics) have improved medical imaging, allowed for rapid pathology testing and characterization of the gut microbiome, allowed superior analysis of biological samples, and provided powerful computational tools to analyze "big data." These are important factors in advancing precision dosing. Our understanding of inter-individual and intra-individual variability in drug response now goes beyond simple patient covariates (e.g. age, gender, weight, etc.) to include the 'hidden' physiological and molecular determinates of pharmacokinetics and pharmacodynamics - organ sizes, blood flows, DMET activities, inflammatory status, gut microbiome, genetics of molecular targets, etc.

Precision medicine requires that the oral dose be titrated based on multiple factors. However, oral dosage forms such as oral tablets are only available in a few selected strengths. The methods and devices disclosed herein permit accurate and flexible dose titrations. For example, the amount of the drug product administered or to be administered to a subject may be determined by characteristics of the patient. Such characteristics may include the subject's genes, environment, and lifestyle as well as age, gender, preexisting conditions, and other relevant characteristics. In such applications, the amount of the drug product administered will typically be a different amount than the amount of drug product in the solid dosage form so as to provide a precise amount of drug product.

Patients are often required to take multiple medications. These medicines often need to be administered one at a time to overcome difficulties patients have with swallowing oral dosage forms such as pills or tablets. This problem is applicable to children. The methods and devices disclosed herein can allow multiple tablets to be simultaneously converted to a single suspension or mixture. This suspension or mixture can be administered such that multiple administrations of individual drugs are avoided. As a result, the patient's compliance with the treatment protocol is enhanced. Combining multiple medications, drug products or active ingredients is also beneficial in a point of care setting. For example, nurses may utilize the disclosed methods and devices to more efficiently administer multiple medications or to administer a precise dose.

Active pharmaceutical ingredient (API) supply is typically very limited during the early clinical development. However, multiple strengths of the drug are required in the single ascending dose (SAD) studies and multiple dose ascending (MAD) studies. The current practice includes: (1) making multiple strengths of the solid dosage forms, (2) 'Powder in bottle' in different strengths (reconstitute before administration), and (3) 'Powder in capsule' in different strengths to accommodate the trial needs. Each of these options requires stability studies to be conducted for at least 3-6 months. Each strength of the tablet must demonstrate adequate stability and dissolution characteristics for the duration of storage which is expensive and time consuming. In addition, for powder in bottle, it is important to demonstrate intimate mixing between the powder and the diluent.

The methods and devices disclosed herein allow one strength of the tablet to be made and different aliquots can be used to meet the needs of the different doses needed in the study. Alternatively, the methods and devices disclosed herein may be used, for example, by adding desired quantity of API powder and mixing with the diluent. Long term stability data is not required since the dose preparation occurs just prior to dosing. The preparation can also be stored in the devices disclosed herein and used the following day.

Typically, clinical trials are conducted with the subject ingesting the solid dosage form followed by 8 ounces of water. This quantity of water ensures that the solid dosage form disintegrates and disperses in the gastric media. This provides the maximum opportunity for the drug to be absorbed. Most absorption of the API occurs in the small intestine (duodenum, jejunum and ileum). The small intestine has the maximum surface area due to the presence of microvilli for absorbing nutrients and drugs. Emulsifying agents such as bile salts are also present in the small intestine. These emulsifying agents aid in the dissolution and subsequent absorption in the small intestine.

In the routine home setting, patients may not be ingesting the drugs with 8 ounces of water. Variability of the time of ingestion and the variability of the gastric content may also influence the disintegration and dissolution of the ingested tablet. The methods and devices disclosed herein transform the solid dosage form to a homogeneous mixture or suspension i.e., the disintegration and dispersion is completed and not influenced by the gastric variability. Therefore, the total dose is available for absorption when the gastric contents transition into the small intestine. This is of special importance for drugs with a narrow therapeutic margin. It avoids any variability due to the gastric physiological variation. It is also possible that overall ingested dose is reduced because of the efficiency of this dose delivery which would result in cost savings.

Many solid dosage forms include a statement instructing the patient or caregiver to crush the tablet and mix with semisolid food or sprinkle the contents of the capsule onto the semisolid food and mix. The methods and devices disclosed herein permit the mixing of the suspended drugs with semisolid food. In some embodiments, the device has a removable bottom half. This permits the use of a spoon to transfer the contents from the device to the oral cavity. Semisolid foods include, but are not limited to, baby formula, apple sauce, avocados, bananas and canned fruits.

The methods and devices disclosed herein allow the patient or caregiver to mimic the churning motion of the human stomach but with greater force and frequency. This permits faster and more intimate contact between the liquid phase and the super disintegrants present in the tablet which results in the fast disintegration of the tablet to form a suspension.

Typically, new drug applications submitted to regulatory authorities are required to include a pediatric investigation plan. Such a plan requires a pediatric friendly formulation for conducting the early development studies. The methods and devices disclosed herein provide an ideal solution for conducting this early development work.

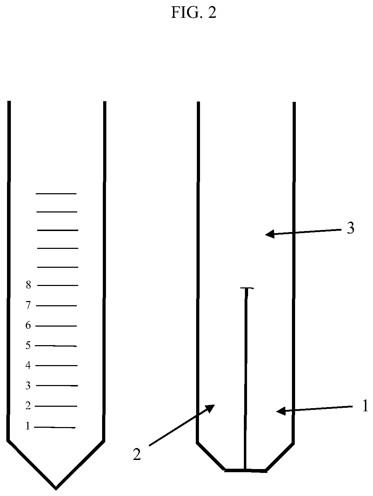

Reference is made to Figure 2 depicting a tubular bag with graduation lines (right) and a two chambered bag (left). The suspension may be mixed then distributed equally between chamber 1 and chamber 2. The two chambered bag has a seam below the hash mark in the middle of the bag. Mixing chamber 3 above the hash mark lacks a center seam and may be used to mix the suspension and assist with evenly distributing the suspension between chamber 1 and chamber 2. The bottom of the bag may be snipped off on the right side to release the contents of Chamber 1 (i.e., half of the suspension) or the left side to release the contents of Chamber 2 (i.e. the other half of the suspension).

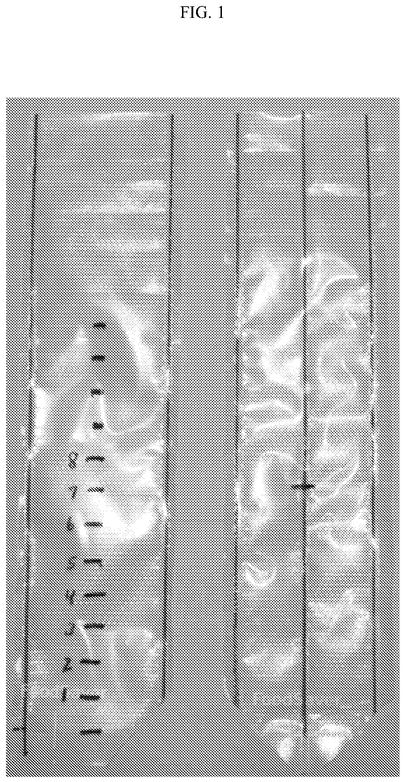

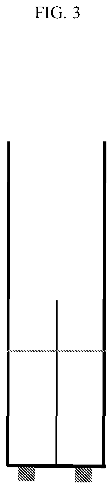

Reference is made to Figure 3 which depicts a two chambered bag similar to Figures 1 and 2. Included is a line showing the level of the suspension in the two lower chambers.

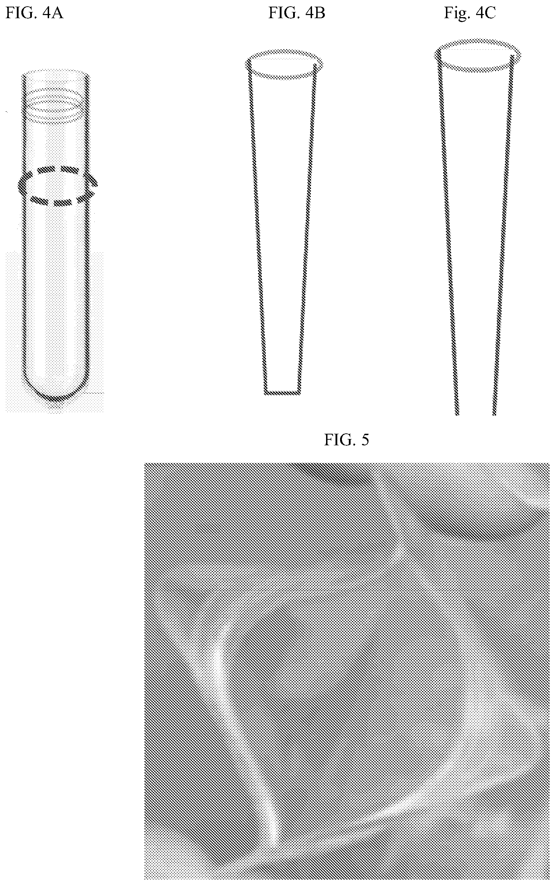

Reference is made to Figure 4A which depicts a container. Figure 4B and 4C depict a firmer second container or a support insert tube for use with the herein disclosed containers. This support tube provides the rigidness to the body of the container. These support tubes facilitate the introduction of the tablet and liquid media into the container. The support tube can preferably have a tapered end that allows for smooth insertion. The support tube may include a cap or a syringe adapter. The support tube may have a closed bottom (Figure 4B) or opened bottom (Figure 4C). The support tube may also be used to facilitate pouring out the contents of the container.

Reference is made to Figure 5 which depicts a squeezable open mouth of a container.

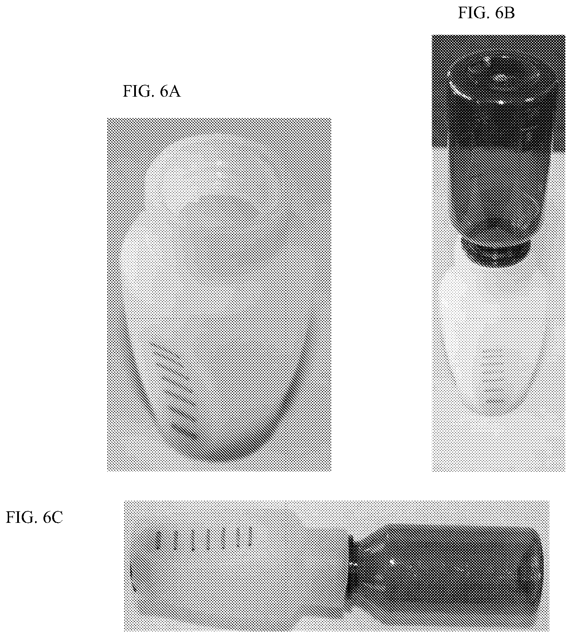

Reference is made to Figure 6A which is a container having a flat bottom, a threaded neck, and graduation marks on the flexible silicone body. The threads of this container may be connected to a prescription bottle (such as a 18mm amber prescription bottle) as exemplified in Figures 6B and 6C.

Reference is made to Figure 7. Figure 7A depicts a container with a middle flat and straight opening. Figure 7B depicts a container with a middle curve and a straight opening. Figure 7C depicts a container with a middle flat and straight opening. Figure 7D depicts a container with straight opening.

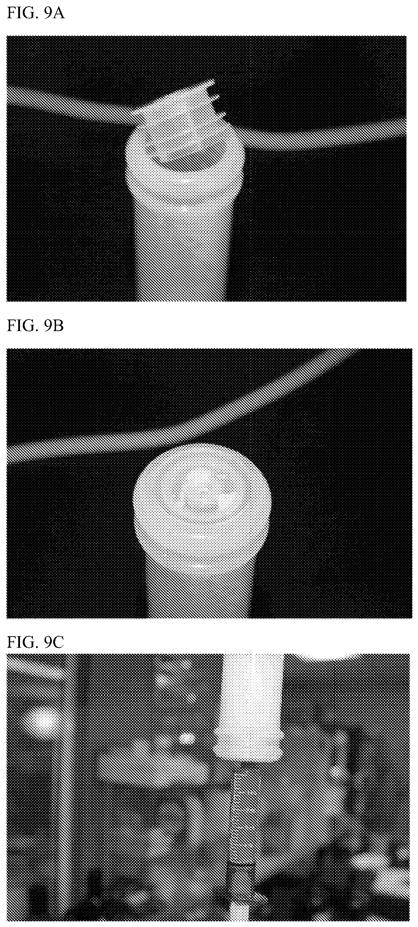

Reference is made to Figure 9 which depicts a syringe adapter and steps of a method for removing the homogeneous mixture or suspension from the container.

The disclosure is further illustrated by the following examples, which are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure and/or scope of the appended claims.

### Example 1

Solid dosage form formulations fail many patients, especially children, because of large tablet or capsule size, poor palatability, and lack of correct dosage strength. The clinical result is often lack of adherence and therapeutic failure. Swallowing difficulties are not solely a pediatric issue. Other patient sub-populations such as the elderly, or those debilitated by stroke might also benefit from formulations specifically designed for children.

Multiple approaches are presently used for breaking, crushing or pulverizing the solid dosage forms. For example, crushing devices, splitting devices, grinding devices and pulverizing devices are available but each have shortcomings.

The problems with the methods commonly used to break, crush or pulverize solid dosage forms include, but are not limited to: (1) the crushed tablets are not contained, and the act of crushing tablets is accompanied with spillage/loss; (2) in most cases the powder resulting from crushing tablets is not homogeneous and contains huge variation in the particle size; (3) if a portion (aliquot) of this powder is administered there is no guarantee of the desired dose being administered; (4) multiple steps are involved including rinsing different pieces of equipment: and (5) the techniques are tedious for daily repetition. Additionally, the active ingredient may not be uniformly distributed within the tablet. (Wagner-Hattler 2020).

In one study, 94 healthy volunteers each split ten 25 mg hydrochlorothiazide tablets. In total, 1752 tablets were manually split and 41.3% deviated from ideal weight by more than 10% and 12.4% deviated by more than 20%. (McDevitt 1998).

In another study, the manipulation of dosage forms among nurses in a hospital setting was studied. The findings included: (1) of the 40 tablet manipulations 25 (62.5%) of the tablets were cut, 12 (30%) dispersed, one (2.5%) crushed, one (2.5%) was broken by hand and there was one (2.5%) manipulation where the tablet was split in half first and then dispersed; (2) the manipulation had to be repeated in three (11.5%) of the 25 tablet manipulations where the tablet had been split, (3) in two cases the tablet crumbled while being split, whilst in the third case the tablet split unevenly; and (4) during a further nine (34.6%) of these manipulations there was also visible powder generated when the tablet was split. (Richey 2013).

In the following example, a commercially available tablet splitter (Walgreens) is used to divide a TBI-223 immediate release (IR) 600mg tablet into quarters to examine the accuracy of such a method. 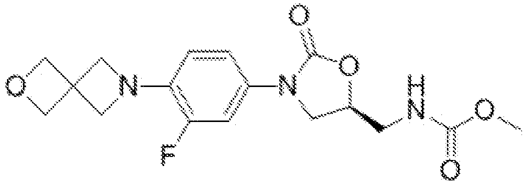 TBI-223

### Results:

The full tablet was initial split into two halves then the first half and second half were split creating four portions. The splitting of the first half resulted in unequal sizes and included significant crushing of tablet material. Splitting the second half resulted in less crushing but the portions were still unequal in size. The four portions were weighed and then the amount of TBI-223 was determined spectrophotometrically by UV absorbance. Weight of split portions is shown in Table 1 below.

Additionally, a fully intact tablet was disintegrated and diluted in 0.1N HCl and filtration through polyethersulfone (PES) syringe filters and analyzed by UV. The intact tablet result was 99.8% of theory (theory = 600mg). This demonstrates that the technique was suitable for the product under evaluation. The same procedure was followed for the four portions from the split tablet and the data is shown in Table 1 which shows very poor reproducibility in both weight and UV tests. The four portions of the tablet varied widely in size, weight, and TBI-223 content as demonstrated by visual observation, weight of the portions, and analysis by UV. The relative standard deviation (RSD) of the four portions was 33.5. This would be an unacceptable way to subdivide and dose because of the differences in the amount of active ingredient between samples.

**Table 1**

| | Weight | Percent of theory |
|---|---|---|
| Intact Tablet | 0.75127g | 99.8 |
| Portion 1 from split tablet | 0.12903 g | 66.9 |
| Portion 2 from split tablet | 0.27129 g | 141.8 |
| Portion 3 from split tablet | 0.19327 g | 101.0 |
| Portion 4 from split tablet | 0.15267 g | 80.6 |

### Example 2

A TBI-223 IR 600mg tablet and 10mL water were combined in a silicone tube and the tablet was completely disintegrated. 14mL of Syrup NF was then added. This is not a volumetric preparation, and the final volume is approximately 25mL.

A placebo was also prepared consisting of 10mL of water and 14mL of Syrup NF.

Four 4mL aliquots of the suspension were removed using 5mL Baxa Oral Dosing Syringes and tested the aliquots for potency by HPLC. Data is shown in Table 2.

It was discovered that the Syrup NF preservative (methyl paraben) was not separated from the TBI-223 peak with this method. Thus, the placebo result (the interference) was subtracted from the sample result. Using this method, the results were a slightly high (106%, likely because it is not a volumetric prepared suspension) but the relative standard deviation (RSD) of the four aliquots tested was excellent (1.1%).

Thus, the inventors have demonstrated that delivery of a suspension prepared from TBI-223 IR tablets is a superior way to deliver a partial dose as compared to simply splitting tablets as in Example 1. The partial doses delivered by suspension has an RSD of 1.1% compared to the split tablet partial doses that had an RSD of 33% in Example 1. Thus, sample reproducibility of partial doses delivered by suspension was excellent.

**Table 2**

| | Volume | Percent of theory |
|---|---|---|
| Portion 1 from dispersed tablet | 4 ml | 107.0 |
| Portion 2 from dispersed tablet | 4 ml | 105.5 |
| Portion 3 from dispersed tablet | 4 ml | 105.1 |
| Portion 4 from dispersed tablet | 4 ml | 107.3 |

### Example 3

A pretomanid 200mg tablet is split as described in Example 1. Each portion was then tested for pretomanid content by HPLC.

### Results:

The four portions of the tablet varied widely in size, weight, and pretomanid content as evidenced by visual observation, weight of the portions, and an analysis by HPLC. The data is shown in Table 3. This is a poor way to subdivide and dose patients because of the variations between patients. The results were similar to what was demonstrated with the TBI-223 tablet in Example 1.

**Table 3**

| | Weight | Percent of theory |
|---|---|---|
| Portion 1 from split tablet | 92.5 g | 90.0 |
| Portion 2 from split tablet | 106.5 g | 102.8 |
| Portion 3 from split tablet | 131.4 g | 128.9 |
| Portion 4 from split tablet | 79.5 g | 77.1 |

### Example 4

The following preparations were prepared. The powder mix used in preparations A, B and C was 0.55g of a medium molecular weight methylcellulose thickener (Methocel^{™} A4C), 2g cane sugar and 2g of an artificially flavored drink mix (Tang^{®} which includes the following ingredients: sugar, fructose, citric acid, ascorbic acid, maltodextrin, calcium phosphate, guar and xanthan gums, sodium acid pyrophosphate, and butylated hydroxyanisole (BHA).

Preparation A:
1. The powder mix was transferred into a silicone screw-cap bag with a TBI-223 IR 600mg tablet and 20mL of water.
2. The bag was capped while gently squeezing to remove some of the headspace air.
3. The contents of the bag were mixed and massaged to wet all the solids.
4. The tablet was continuously mixed and massaged by hand until the tablet material was completely disintegrated and a uniform suspension obtained. The tablet material was completely disintegrated in about 14 minutes.

Preparation B:
1. The powder mix was transferred into a silicone screw-cap bag with a TBI-223 IR 600mg tablet and 20mL of water.
2. The bag was capped while gently squeezing to remove some of the headspace air.
3. The contents of the bag were mixed and massaged to wet all the solids.
4. Using a spoon (any hard object may be used) the tablet was crushed through the bag with a downward and grinding forceful motion.
5. The resulting mixture was mixed and massaged by hand until a uniform suspension was obtained, which occurred in about 6-7 minutes.

Preparation C:
1. The powder mix was transferred into a silicone screw-cap bag with a TBI-223 IR 600mg tablet and 20mL of water.
2. The bag was capped while gently squeezing to remove some of the headspace air.
3. The contents of the bag were mixed and massaged to wet all the solids.
4. The mixture was left idle for 15 minutes.
5. The remaining tablet was crushed with a spoon through the bag with a downward and grinding forceful motion.
6. The resulting mixture was mixed and massaged by hand until a uniform suspension was obtained, in about 4-5 minutes.

The fastest technique among preparations A, B and C was to crush the tablet through the silicone bag using a spoon or other hard object which can be accomplished in less than 10 minutes.

The powder mix used in preparations D, E, F and H was 0.27g of a medium molecular weight methylcellulose thickener (Methocel^{™} A4C), 2g cane sugar and 2g of an artificially flavored drink mix (Tang^{®}).

Preparation D:
1. The powder mix was transferred into a silicone screw-cap bag with a TBI-223 IR 600mg tablet and 20mL of water.
2. The contents of the bag were mixed and massaged to wet all the solids.
3. Mixture was left idle for about 4-5 minutes, then the tablet was briefly mixed and massaged by hand, but the tablet was not disintegrated. After leaving idle for another 4-5 minutes, the tablet is still not disintegrated. It is not until approximately 23 minutes in total that the tablet is completely disintegrated.

Preparation E:
1. A TBI-223 IR 600mg tablet and 20mL of water is added into a silicone screw-cap bag.
2. The contents of the bag were mixed and massaged to wet all the solids.
3. The mixture was left idle for about 4-5 minutes.
4. The tablet were mixed and massaged by hand until the tablet material is completely disintegrated and a uniform suspension was obtained. The tablet material is completely disintegrated in less than 30 seconds.
5. The power mix was added and mixed and massaged with the tablet pieces by hand until a uniform suspension is obtained, about 1 minute.

Preparation F:
1. The powder mix was transferred into a silicone screw-cap bag with a TBI-223 IR 600mg tablet and 20mL of water.
2. The contents of the bag were mixed and massaged to wet all the solids.
3. The mixture was left to sit for about 30 minutes, after briefly mixing and massaging the tablet pieces by hand, the tablet was completely disintegrated.

Preparation G:
1. A 25 mg USP Chlorthalidone tablet and 8mL of water is added to an elongated nipple and mixed. A disintegrated tablet mix is formed almost immediately.

Preparation H:
1. A TBI-223 IR 600mg tablet and 20mL of water was added into a silicone screw-cap bag.
2. The mixture was left idle for about 3 minutes and the tablet pieces were then briefly mixed and massaged by hand for 30 seconds, and the mixture was left idle for another 2 minutes. After a briefly mixing and massaging, the tablet is completely disintegrated and a uniform suspension is obtained after about 5 minutes.
3. The powder mix was added and contents were mixed and massaged by hand until a uniform suspension is obtained, about 2 minutes.

In each of the above preparations, a dose of the mixed suspension may be removed using a dosing spoon or syringe.

The above results demonstrate that dispersing the solid dosage form thereby forming a homogeneous suspension prior to adding the powder mix, as exemplified by Preparation E, is preferred to adding the powder mix before the solid dosage form is dispersed as exemplified by Preparation D.

### Example 5

To a disposable bag as shown in the left side of Figure 1 or Figure 2 is added a tablet and 10 ml of water. The contents of the disposable bag are mechanically mixed by hand, for example, by squeezing the dosage form so as to break up the tablet. The graduation lines are used to measure an aliquot of half the homogeneous suspension (about 5 ml) which may be administered to a patient. Alternatively, an amount of water may be added such that the total volume of the contents added to the bag is 10 ml, and 5 ml of the homogeneous suspension may be administered to the patient.

### Example 6

To a disposable bag as shown in the left side of Figure 1 or Figure 2 is added a tablet, and a powder blend consisting of a methylcellulose thickener and sugar and artificially flavoring and 10 ml of water. The contents of the disposable bag are mechanically mixed by hand, for example, by squeezing the dosage form so as to break up the dosage form to form a homogeneous suspension. The graduation lines are used to measure an aliquot of half the homogeneous suspension (about 5ml) which may be administered to a patient. Alternatively, an amount of water may be added such that the total volume of the contents added to the bag is 10 ml, and 5 ml of the homogeneous suspension may be administered to the patient.

### Example 7

To a disposable bag as shown in the right side of Figure 1 or Figure 2 is added a tablet and 10 ml of water. The contents of the disposable bag are mechanically mixed by hand, for example by squeezing the dosage form so as to break up the tablet. The bottom portion of the bag has two identical chambers. The suspension is manipulated so that each chamber contains the same volume so that delivery from one side represents one half of the total. The portion of the homogeneous suspension in one chamber may be administered to a patient.

### Example 8

To a disposable bag as shown in the right side of Figure 1 or Figure 2 is added a tablet, and a powder blend consisting of a methylcellulose thickener and sugar and artificially flavoring and 10 ml of water. The contents of the disposable bag are mechanically mixed by hand, for example, by squeezing the dosage form so as to break up the dosage form to form a homogeneous suspension. The bottom portion of the bag has two identical chambers. The suspension is manipulated so that each chamber contains the same volume so that delivery from one side represents one half of the total. The portion of the homogeneous suspension in one chamber may be administered to a patient.

### Example 9

A dosing aid is used which comprises a thin, pliable, flexible round bottom bag having an opening at top. A rigid tapered tube having at least 2 openings is also used. The rigid tapered tube is inserted into the dosing aid. The drug product and the liquid are then inserted into the opening of the rigid tapered tube. The rigid tapered tube has an opening on the top and bottom, similar to the housing shown in Figure 4C. Thus, when the rigid tapered tube is removed from the dosing aid, substantially all of the drug product and the liquid remain in the dosing aid. The drug product and the liquid are mixed in the dosing aid to form a homogeneous suspension.

### Example 10

Certain patient populations may have difficulty disintegrating and dispersing the tablets by physical manipulation without assistance of an electronic device. Therefore, electronic devices to aid this process were evaluated. The amount of time required to disintegrate and disperse the tablet in a liquid medium using manual physical manipulation, and machine physical manipulation were calculated and compared as described below.

A. Container with human hand.

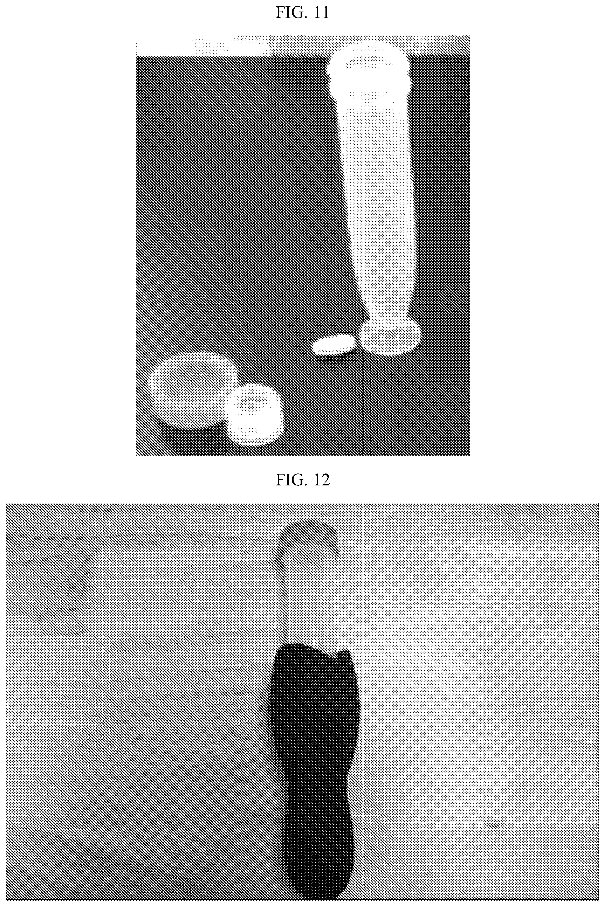

A pretomanid 200 mg tablet was added to the container depicted in Figure 11 followed by 10 mL of water. This container is made of a flexible material and is similar in shape to the container depicted in Figure 7D. The tablet and water in the device were mixed by shaking, pressing, rubbing, and massaging the tablet between the thumb and fingers of a human. The tablet disintegrated and dispersed homogeneously in water after approximately 2 minutes.

### B. Vibration device

An electronic vibrator Bombombda, model number S10100-50 was utilized for providing the vibrational forces for dispersing the tablet. To the same container depicted in part A was added a pretomanid 200 mg tablet followed by 10 mL of water. The container was inserted into the vibrator as shown in Figure 12 and the vibrator was turned on. The frequency of vibrations was far greater that that could be accomplished by manual shaking.

It was surprisingly discovered that the tablets failed to disintegrate and disperse even after 3 minutes of mixing with the vibrator.

### C. Vortex Shaker

To the same container depicted in part A above was added a pretomanid 200 mg tablet and 10 mL of water. The container was mounted on a vortex shaker (Thermolyne Maxi Mix, Thermo Fisher Scientific). This mixing mechanisms of the vortex shaker include vibrations, shaking, surface erosion, shearing, shaking and rubbing of the water and tablet. A homogeneous suspension was prepared after 2.5 minutes. It was surprisingly discovered that an efficient device for dispersing a tablet would require multiple mixing mechanisms.

### Example 11

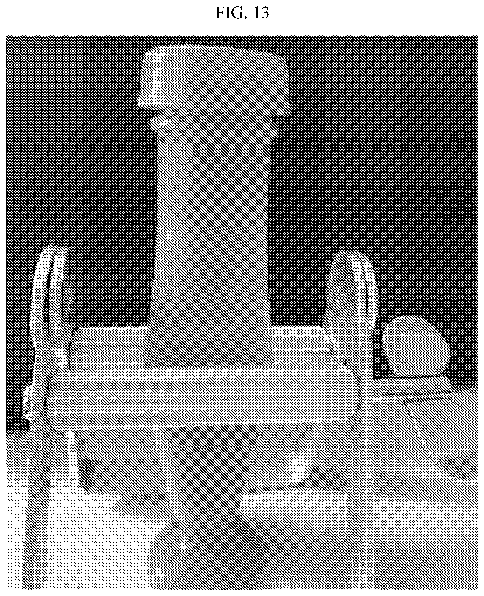

A device comprising two rollers as shown in Figure 13 is used. These rollers turn simultaneously but in opposite directions. The lever can be turned in clockwise and counterclockwise directions to place mechanical force on the container. This allows for homogeneous mixing of a tablet.

### Example 12

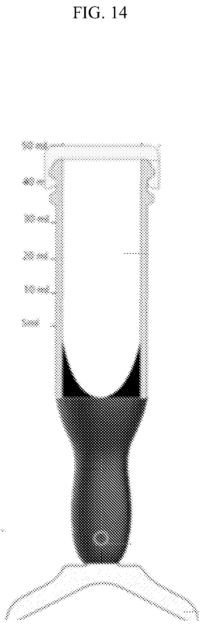

To a container is added a tablet, and a powder blend consisting of a methylcellulose thickener and sugar and artificially flavoring and 10 ml of water. The container is attached to an electric machine which provides one or more of a vibrational force, a shaking force, a mechanical force, a rubbing force, and/or mechanical pressure to the flexible section of the container. An example of the container and electric machine is depicted in Figure 14. This machine includes a cap, a silicon chamber for tablet dispersion, a vibrating unit and a stand.

### Example 13

Powder blends were prepared as described in Table 4.

| Table 4 | | | | | | | | | | | | | | | |
|---|---|---|---|---|---|---|---|---|---|---|---|---|---|---|---|
| | Concentration | 1 | 2 | 3 | 4 | 5 | 6 | 7 | 8 | 9 | 10 | 11 | 12 | 13 | 14 |
| **Suspending agent / Thickener** | | | | | | | | | | | | | | | |
| T1 (Gum combination: Guar Gum, Gum Acacia, Xanthan Gum) (cold water soluble) | 0.1-0.5% | | | | | | | x | | | | | | | x |
| T2 (Microcrystalline cellulose) | 2% | x | | | | | | | | | | | | x | |
| T3 (Gum acacia) | 0.2% | | x | | | | | | | | | | x | | |
| T4 (Guar gum) (cold water soluble) | 0.1-0.5% | | | x | | | | | | | | x | | | |
| T5 (Xanthan gum) | 5% | | | | x | | | | | | x | | | | |
| T6 (Modified corn starch) | 2.5% | | | | | x | | | | x | | | | | |
| T7 (Maltodextrin) | 4% | | | | | | x | | x | | | | | | |

| **Bitter blocker** | | | | | | | | | | | | | | | |
|---|---|---|---|---|---|---|---|---|---|---|---|---|---|---|---|
| B1 (ClearTaste) | 0.01-1% | x | | | | x | | | | | | x | | | |
| B2 (TruClear) | 5% | | x | | | | x | | | | x | | x | | |
| B3 (Bitterend) | 0.002 - 0.02% | | | x | | | | x | | x | | | | x | |
| B4 (Bitter blocker N) | 0.002 - 0.02% | | | | x | | | | x | | | | | | x |

| **Flavor** | | | | | | | | | | | | | | | |
|---|---|---|---|---|---|---|---|---|---|---|---|---|---|---|---|
| F0 (no F) | | | | | | | | | | | | | | | |
| F1 (Pineapple) | 0.06% | x | | | | | x | | | | | | | | x |
| F2 (Orange) | 0.2% | | x | | | | | x | | | | | | x | |
| F3 (Caramel) | 1% | | | x | | | | | x | | | | x | | |
| F4 (Apple) | 0.5% | | | | x | | | | | x | | x | | | |
| F5 (Bubble gum) | 0.05-0.4% | | | | | x | | | | | x | | | | |
| **Sweetener** | | | | | | | | | | | | | | | |
| S1 (Sugar or Sucrose) | 50% | x | | | | x | | | | | | x | | | |
| S2 (Saccharin) | 0.07% | | x | | | | x | | | | x | | x | | |
| S3 (Sucralose) | 0.01% | | | x | | | | x | | x | | | | x | |
| S4 (Aspartame) | 0.015% | | | | x | | | | x | | | | | | x |

| **Antimicrobial system** | | | | | | | | | | | | | | | |
|---|---|---|---|---|---|---|---|---|---|---|---|---|---|---|---|
| A1 (Methyl paraben and potassium sorbate) | 0.04% paraben and 0.08% sorbate | x | | x | | x | | x | | x | | x | | x | |

### REFERENCES

McDevitt JT, Gurst AH, Chen Y. Accuracy of tablet splitting. Pharmacotherapy. 1998;18(1):193-197.

Richey, Roberta H et al. "Manipulation of drugs to achieve the required dose is intrinsic to pediatric practice but is not supported by guidelines or evidence." BMC pediatrics vol. 13 81. 21 May 2013.

Sharma N, Pahuja S and Sharma N: Immediate release tablets: a review. Int J Pharm Sci & Res 2019; 10(8): 3607-18. doi: 10.13040/ IJPSR.0975-8232.10(8).3607-18.

Wagner-Hattler L, Québatte G, Keiser J, et al. Study of drug particle distributions within mini-tablets using synchrotron X-ray microtomography and superpixel image clustering. Int J Pharm. 2020;573:118827.

U.S. Department of Health and Human Services Food and Drug Administration Center for Drug Evaluation and Research (CDER) "Guidance for Industry Orally Disintegrating Tablets" December 2008.

It is to be understood that the invention is not limited to the particular embodiments of the invention described above, as variations of the particular embodiments may be made and still fall within the scope of the appended claims.

The invention will be further described, without limitation, by the following numbered paragraphs:
1) A method of preparing a homogeneous mixture from a solid dosage form comprising
   a) obtaining a solid dosage form comprising a drug product,
   b) adding the solid dosage form to a container,
   c) adding a liquid to the container,
   d) mixing the solid dosage form with the liquid to disperse, disintegrate, suspend, and/or dissolve the solid dosage form thereby forming a homogeneous mixture;
   wherein
   the container comprises at least one flexible section, and
   the method is performed at a point of care of a subject, and/or
   one or more additional solids dosage forms are added to the container, and/or wherein the homogeneous mixture is intended to be administered to a subject and
      (i) the subject is a human or a non-human animal,
      (ii) the amount of the drug product administered or to be administered to the subject is determined by characteristics of the subject,
      (iii) the subject is participating in a clinical trial,
      (iv) the subject is a pediatric subject, or
      (v) the subject is a geriatric subject.
2) The method of paragraph 1, wherein the homogeneous mixture is a homogeneous suspension.
3) The method of paragraph 1 or 2, wherein the solid dosage form is dispersed in step d).
4) The method of any one of paragraphs 1-3, wherein the liquid is water, optionally 1-50 ml, 1-20 ml, 1-10 ml, or 1-5 ml of water.
5) The method of any one of paragraphs 1-4, wherein the drug product is an immediate release (IR) tablet, an orally dissolving tablet (ODT), or a dispersible tablet (DT).
6) The method of any one of paragraphs 1-5, wherein the solid dosage form comprises a disintegrant.
7) The method of any one of paragraphs 1-6, wherein the mixing comprises applying a force to the container.
8) The method of paragraph 7, wherein the force is a vibrational force, a shaking force, a mechanical force, a rubbing force, and/or mechanical pressure.
9) The method of paragraph 7 or 8, wherein the force is applied to the flexible section of the container.
10) The method of any one of paragraphs 7-9, wherein the force is applied by a human, optionally wherein the human is the subject.
11) The method of any one of paragraphs 7-9, wherein the force is applied by a machine.
12) The method of paragraph 11, wherein the machine comprises a shaking mechanism, a vibrating mechanism, a mechanism to provide mechanical force, a mechanism to provide rubbing force, and/or a mechanism to provide mechanical pressure.
13) The method of paragraph 11 or 12, wherein the force is at least a mechanical force and a vibrational force.
14) The method of any one of paragraphs 1-13, wherein the container comprises graduations, optionally, wherein the graduations are in increments of 0.5 ml or less, 0.75 ml or less, 1.0 ml or less, 1.5 ml or less, 2.0 ml or less, 3.0 ml or less, 4.0 ml or less, 5.0 ml or less, or 10 ml or less, 0.5 ml, 0.75 ml, 1.0 ml, 1.5 ml, 2.0 ml, 3.0 ml, 4.0 ml, 5.0 ml or 10 ml.
15) The method of any one of paragraphs 1-14, wherein the container comprises a first chamber, a second chamber and a mixing chamber, wherein the first chamber and second chamber have equal volumes and the first chamber is in connection with the mixing chamber and the second chamber is in connection with the mixing chamber.
16) The method of paragraph 15, further comprising distributing the homogeneous mixture such that equal volumes are present in the first chamber and the second chamber.
17) The method of any one of paragraphs 15-16, further comprising administering the homogeneous mixture from the first chamber to a patient in need thereof or transferring the homogeneous mixture from the first chamber.
18) The method of any one of paragraphs 1-17, wherein the solid dosage form is a solid oral dosage form.
19) The method of any one of paragraphs 1-18, wherein the at least one flexible section comprises a flexible film, for example, a polyethylene film or silicone film.
20) The method of any one of paragraphs 1-19, wherein the container comprises an opening on a top end.
21) The method of paragraph 20, wherein the opening is defined by opposite side edges capable of being reversibly coupled together.
22) The method of paragraph 21, wherein the opposed side edges meet at two corners and the edges are a firmer flexible film such that applying pressure simultaneously to both edges results in a larger opening forming while such pressure is applied.
23) The method of any one of paragraphs 1-22, wherein the container is free of rigid sections.
24) The method of any one of paragraphs 1-23, wherein at least one exterior surface of the container comprises a flat surface.
25) The method of any one of paragraphs 1-24, wherein the container comprises an exterior bottom end which comprises a means for standing upright, optionally wherein the means for standing upright is a suction cup or a stabilizing base.
26) The method of any one of paragraphs 1-25, wherein the container comprises an internal bottom end comprising a tapered bottom, hemispherical bottom, or conical bottom.
27) The method of any one of paragraphs 1-26, wherein the container comprises a handle.
28) The method of any one of paragraphs 1-27, wherein the container comprises at least two parts, a first part having a first opening and a second opening, and a second part having a third opening, wherein the second opening is capable of attaching to and forming a seal with the third opening to form the container.
29) The method of any one of paragraphs 1-28, wherein the container comprises an inner side and an outer side, wherein the inner side and/or the outer side comprises bumps, a coarse texture, protrusions, rings, spirals, dots, raised surfaces and/or ridges.
30) The method of any one of paragraphs 1-29, wherein the container is colored or opaque to prevent light from penetrating, or the container is non-translucent, optionally, wherein the light is ultraviolet light.
31) The method of any one of paragraphs 1-30, wherein the container comprises a means for reversibly enclosing and sealing the container.
32) The method of any one of paragraphs 1-31, wherein the container comprises an opening and a cap capable of reversibly closing the opening.
33) The method of paragraph 32, wherein the cap is a threaded cap and the opening is a threaded opening such that the cap and opening form a seal when the container is closed.
34) The method of any one of paragraphs 32-33, wherein the cap comprises a spoon.
35) The method of any one of paragraphs 32-33, wherein the cap is a syringe adapter.
36) The method of any one of paragraphs 20-35, wherein the method comprises attaching a syringe adapter to the opening.
37) The method of any one of paragraphs 20, 35-36, wherein the method comprises attaching a syringe to the syringe adapter and extracting an amount of the homogeneous mixture from the container.
38) The method of any one of paragraphs 1-37, wherein the container comprises a thin pliable polymer, optionally, polyethylene, polyethylene terephthalate, or polypropylene.
39) The method of any one of paragraphs 1-38, further comprising inserting a rigid support tube into the container.
40) The method of any one of paragraphs 1-39, further comprising inserting the solid dosage form and the liquid into the rigid support tube and subsequently removing the rigid support tube from the container prior to mixing the solid dosage form.
41) The method of any one of paragraphs 1-40, further comprising forming an enclosed and sealed container.
42) The method of any one of paragraphs 1-41, wherein the container and cap each independently consist of one or more inert materials which do not react with the drug product.
43) The method of any one of paragraphs 1-42, further comprising separating an aliquot of the homogeneous mixture.
44) The method of any one of paragraphs 1-43, further comprising administering the homogeneous mixture or an aliquot of the homogeneous mixture to a subject in need thereof, thereby treating the subject.
45) The method of paragraph 44, wherein the subject is a pediatric subject or a subject afflicted with dysphagia.
46) The method of paragraph 44 or 45, wherein the subject is afflicted with a microbial infection.
47) The method of paragraph 46, wherein the microbial infection is caused by Mycobacterium tuberculosis.
48) The method of any one of paragraphs 1-47, further comprising adding a powder blend to the container, wherein the powder blend comprises a thickener.
49) The method of paragraph 48, wherein the powder blend is added to the container after the homogeneous mixture is formed.
50) A powder blend comprising a thickener.
51) A powder blend of paragraph 50, wherein the thickener is methylcellulose, a medium molecular weight methylcellulose, or a thickener which yields a viscosity of 400 cP at 2% in water.
52) A powder blend according to paragraph 50 or 51, wherein the powder blend further comprises a thickener, sugar or a flavoring agent.
53) A container for use in transforming a solid dosage form into a homogeneous mixture at a point of care of a subject, comprising
   a) at least one flexible section,
   b) a means for reversibly enclosing and sealing the container, and
   c) graduations.
54) The container of paragraph 53, wherein the graduations are in increments of 0.5 ml or less, 0.75 ml or less, 1.0 ml or less, 1.5 ml or less, 2.0 ml or less, 3.0 ml or less, 4.0 ml or less, 5.0 ml or less, or 10 ml or less, 0.5 ml, 0.75 ml, 1.0 ml, 1.5 ml, 2.0 ml, 3.0 ml, 4.0 ml, 5.0 ml or 10 ml.

## Claims

1. A method of preparing a homogeneous mixture from a solid dosage form comprising
a) obtaining a solid dosage form comprising a drug product,
b) adding the solid dosage form to a container,
c) adding a liquid to the container,
d) mixing the solid dosage form with the liquid to disperse, disintegrate, suspend, and/or dissolve the solid dosage form thereby creating a homogeneous mixture;
wherein the container comprises at least one flexible section and the method is performed at a point of care of a subject.

2. The method of claim 1, wherein the liquid is water, optionally 1-50 ml, 1-20 ml, 1-10 ml, or 1-5 ml of water.

3. The method of claim 1, wherein the drug product is an immediate release (IR) tablet, an orally dissolving tablet (ODT), or a dispersible tablet (DT).

4. The method of claim 1, wherein the solid dosage form comprises a disintegrant.

5. The method of claim 1, wherein the mixing comprises applying a force to the container.

6. The method of claim 1, wherein the container comprises graduations, optionally, wherein the graduations are in increments of 0.5 ml or less, 0.75 ml or less, 1.0 ml or less, 1.5 ml or less, 2.0 ml or less, 3.0 ml or less, 4.0 ml or less, 5.0 ml or less, or 10 ml or less, 0.5 ml, 0.75 ml, 1.0 ml, 1.5 ml, 2.0 ml, 3.0 ml, 4.0 ml, 5.0 ml or 10 ml.

7. The method of claim 1, wherein the container comprises a first chamber, a second chamber and a mixing chamber, wherein the first chamber and second chamber have equal volumes and the first chamber is in connection with the mixing chamber and the second chamber is in connection with the mixing chamber.

8. The method of claim 7, further comprising distributing the homogeneous mixture such that equal volumes are present in the first chamber and the second chamber.

9. The method of claim 1, wherein the at least one flexible section comprises a flexible film, for example, a polyethylene film or silicone film.

10. The method of claim 1, wherein the container comprises an opening on a top end.

11. The method of claim 10, wherein the opening is defined by opposite side edges capable of being reversibly coupled together.

12. The method of claim 11, wherein the opposed side edges meet at two corners and the edges are a firmer flexible film such that applying pressure simultaneously to both edges results in a larger opening forming while such pressure is applied.

13. The method of claim 1, wherein the container is free of rigid sections.

14. The method of claim 1, wherein the container comprises an exterior bottom end which comprises a means for standing upright, optionally wherein the means for standing upright is a suction cup or a stabilizing base.

15. The method of claim 1, wherein the container comprises at least two parts, a first part having a first opening and a second opening, and a second part having a third opening, wherein the second opening is capable of attaching to and forming a seal with the third opening to form the container.
